# EUROPEAN PATENT SPECIFICATION

(11) **EP 3 616 596 B1**
(45) Date of publication and mention of the grant of the patent: **30.04.2025**
(21) Application number: 19204605.0
(22) Date of filing: 23.05.2016
(51) Int. Cl.: A61B 1/00, A61B 1/04, A61B 1/06, G01N 21/64, G02B 21/00, G02B 23/24, G06T 5/50, A61B 8/12, G06T 7/00, G02B 21/16, A61B 8/06, A61B 8/08, A61B 8/00, A61B 6/00, A61B 6/50

(54) **MICROSCOPE AND METHOD USING PSEUDO-COLOR IMAGE DATA**
MIKROSKOP UND VERFAHREN MIT PSEUDO-FARBBILDDATEN
MICROSCOPE ET PROCÉDÉ UTILISANT DES DONNÉES D'IMAGE EN PSEUDO-COULEUR

(43) Date of publication of application: 04.03.2020
(62) Divisional of application: 16170769.0
(73) Proprietor: Leica Instruments (Singapore) Pte. Ltd., 608924 Singapore (SG)
(72) Inventor: THEMELIS, George, 88131 Lindau (DE); GESSAT, Michael, 8280 Kreuzlinge (CH)
(74) Representative: Grünecker Patent- und Rechtsanwälte PartG mbB

(56) References cited:
- EP-A1- 1 685 795
- EP-A1- 2 932 882
- US-A1- 2005 203 420
- US-A1- 2011 275 932

## Description

The invention relates to a microscope and a method for displaying medical images.

In US 2005/0203420, a method for the merged display of first image information captured using a first imaging device with second information captured using a second imaging device is shown, whereby said image information relates to the same area of examination in a hollow organ such as a prostate gland, bowel, gullet, bronchial tubes or vascular system. First, two-dimensional images are obtained from the area under examination using a continuous controlled movement of a medical instrument such as a catheter, an endoscope, a laparoscope or a colonoscope. The individual images are compiled into a three-dimensional fluorescence data record. The fluorescence data record is then registered with a three-dimensional data record, for example, taken by an MR or CT, 3D angiographic or 3D ultrasound system.

In EP 1 685 795 A1, an apparatus is described, which acquires tomographic images at depths from zero to several millimeters from a surface of a region. A probe is inserted through a forceps channel of an endoscope and rotated along its longitudinal axis. Thus, tomographic images are obtained, again from a hollow organ. The probe allows the acquisition of optical tomographic images generated using optical coherence tomography, ultrasound-modulated-light tomography and ultrasound-modulated-fluorescence tomography as well as ultrasonic tomography.

US 2011/0275932 A1 discloses a tomographic fluorescent imaging device for imaging fluorophores in biological tissues has a scanned laser for scanning the tissue and a camera for receiving light from the biological tissue at an angle to the beam at a second wavelength ten or more nanometers greater in wavelength than the wavelength of the laser. Images are obtained at each of several positions of the beam. An image processing system receives the series of images, models a path of the beam through the tissue, and determines depth of fluorophore in tissue from intersections of the modeled path of the beam and the path of the received light.

It is known in the prior art to inject a fluorophore into tissue that is observed by an optical imaging device such as a microscope or an endoscope. The fluorophore is configured to mark only specific tissue. For example, the fluorophore may be adapted to remain in the blood stream only during the observation time so that only blood vessels are marked by the fluorophore. Another fluorophore may be provided in the form of a precursor material, which reacts only with tumor cells to produce a fluorophore which only marks cancer. Thus, fluorescence imaging can provide useful diagnostic information. However, fluorescence typically does not provide anatomical information, i.e. tissue color appearance. Conversely, visible-light image provides anatomical information but not fluorescence diagnostic information. Pseudocolor is a convenient way to present in a single image the information of those two different image types: visible-light and fluorescence. It is known in the prior art to superpose or merge the visible-light images and the fluorescence images.

The use of such an image superposition device and method greatly facilitates diagnosis and surgery. However, there is a need for further improving the technology thus to further facilitate diagnosis and surgery.

It is therefore the object of the present invention to provide a device and method, which further improves the existing devices and methods.

This object is solved by the subject-matter of the independent claims.

Preferred embodiments are defined by the dependent claims.

This goal is achieved by a microscope according to claim 1 which comprises, inter alia, an input interface and an output interface, the input interface being configured to receive subsequent sets of input image data and subsequent sets of auxiliary input data, the output interface being configured to output subsequent sets of output image data at an output data rate, the image processor being connected to the input interface for receiving the sets of input image data and auxiliary input data and to the output interface for outputting the output image data, the microscope further comprising an image processor wherein the image processor is connected to the input interface for receiving the subsequent sets of the input image data and the auxiliary input data, and to the output interface for outputting the subsequent sets of the output image data , the image processor further being configured to generate, in real time, subsequent sets of pseudo-color image data depending on a content of at least one of the auxiliary input data and the input image data, wherein the output image data are three-dimensional and wherein the image processor is configured to generate pseudo-color image data at different depth layers of the three-dimensional output image data depending on the content of the auxiliary input data.

Another device not forming part of the invention is a medical observation device which comprises an input interface, an image processor and an output interface, the input interface being configured to receive subsequent sets of input image data and subsequent sets of auxiliary input data, the output interface being configured to output subsequent sets of output image data at an output data rate, the image processor being connected to the input interface for receiving the sets of input image data and auxiliary input data and to the output interface for outputting the output image data, the image processor being further configured to generate subsequent sets of pseudo-color image data depending on a content of at least one of the auxiliary input data and the input image data and to generate at least one synthetic pseudo-color pattern field within the pseudo-color image data depending on the content of at least one of the input image data, the auxiliary input data and the pseudo-color image data, the pseudo-color pattern field comprising at least one of a temporally and a spatially changing pattern, and the image processor being configured to merge the pseudo-color image data and at least one section of the input image data to generate the output image data containing the pseudo-color pattern field.

The objective of the invention is also achieved by a method for displaying microscope images according to claim 12, comprising, inter alia, the steps of receiving subsequent sets of input image data, receiving subsequent sets of auxiliary input data, generating subsequent sets of pseudo-color image data in real time depending on the content of at least one of the auxiliary input data and the input image data, merging the pseudo-color image data with the input image data to obtain three-dimensional output image data, wherein the pseudo-color image data are generated at different depth layers of the three-dimensional output image data depending on the content of the auxiliary input data, and displaying the output image data at an output data rate.

Another method not forming part of the invetion for displaying medical images comprises the steps of receiving subsequent sets of input image data, receiving subsequent sets of auxiliary input data, generating subsequent sets of pseudo-color image data depending on the content of at least one of the auxiliary input data and the input image data, generating at least one coherent pattern field within the pseudo-color image data depending on the content of at least one of the input image data, the auxiliary input data and the pseudo-color image data, the pattern field having at least one of a spatial and a temporal modulation, merging the pseudo-color image data with the input image data to obtain output image data, and displaying the output image data with the pseudo-color pattern field.

In the following, further aspects of the invention are described. Each of the further aspects described herein below, has its own advantage and technical effect.

According to one aspect, the image processor may be configured to generate a synthetic pseudo-color pattern field within the pseudo-color image data depending on the content of at least one of the input image data, the auxiliary input data and the pseudo-color image data, the pseudo-color pattern field comprising at least one of a temporally and/or spatially changing pseudo-color pattern, and the image processor being configured to merge the pseudo-color image data and at least one section of the input image data to generate the output image data containing the pseudo-color pattern field.

The use of the temporally and/or spatially modulated pseudo-color pattern allows the merging of further data in particular representing additional data sources to the output image. Further, the use of a pseudo-color pattern allows visualizing data in the output image data that would be otherwise be imperceptible in the pseudo-color image.

As the pattern field is both of pseudo-color and synthetic, it is immediately distinct from the input image, which is preferably used as the background in the output image data and based on visible-light image data.

In the context of this invention, a pattern designates a discernible regularity, in which elements repeat in a predictable manner. The regularity and predictability means that the pattern is not reliant on human cognitive processes. Rather, the pattern can be automatically discerned and discovered using pattern recognition algorithms. A pseudo-color is a color which does not occur naturally in that context, such as a neon color in biological tissues. A color in the context of this disclosure is marked as a particular combination of wavelengths of light and thus independent of human cognitive processes.

For example, the pattern field may be filled with a spatial pattern such as a hatching or with a regular repetition in time and/or space of a template. A template may, in a pattern having spatial modulation, consist of geometric forms such as dots, tiles, symbols and/or pictures. In a pattern having temporal modulation, the pattern consists of a regular repetition of a set of different images over time. A typical example of such a pattern is a hatching or a bitmap template of simple geometric forms such as circles, polygons and/or waves.

In a pattern having both temporal and spatial modulation, different patterns having spatial modulation are repeatedly displayed. The subsequent patterns in a temporally modulated pattern may be similar to each other, e.g. spatially displaced relative to each other but otherwise identical, to represent a direction of motion. For example, subsequent hatchings of a temporally modulated pattern within one cycle of the variation rate may all be shifted geometrically with respect to each other by the same amount. This can be used to produce a temporally modulated pattern which indicates a motion direction. A propagating wave pattern may, e.g. be used to indicate the direction of a fluid flow, such as a blood or lymph flow.

The pseudo-color pattern preferably extends over at least one coherent or contiguous section of the pseudo-color image data and thus forms a pseudo-color pattern field. Within a pseudo-color pattern field, the content of at least one of the image input data and the auxiliary input data satisfies preferably the same condition. For example, if the content of the auxiliary input data and/or the pseudo-color image data exceeds an upper threshold, this may represent saturation in the data. These data may then be represented by the pattern field so that they are marked as being unreliable. The same approach can be used in connection with a lower threshold, which may represent data which are below a sensitivity threshold and thus may be considered unreliable. An example of a temporal modulated pattern may be a pseudo-color image or a coherent part thereof, which is filled with a pseudo-color and in which the pseudo-color is switched on and off, or changes at least one of brightness, hue and saturation according to the variation rate.

A temporally modulated pattern may have a variation rate in which a cyclic repetition of alternating patterns occurs. In order to be clearly visible, the variation rate should be smaller than the flicker fusion rate, i.e. in particular be smaller than 25 Hz.

According to the invention, the image processor is configured to determine the variation rate depending on the content of at least one of the input image data and the auxiliary input data. The variation rate may be automatically varied by the image processor over time. In particular, the variation over time may be dependent on the content of at least one of the input image data, the auxiliary input data and the pseudo-color image data. If, for example, the auxiliary data are ultrasound data, the variation rate may depend on the momentary flow velocity, or on a frequency and/or amplitude of velocity changes, e.g. caused by a pulse in a blood flow.

If the pseudo-color pattern field is a temporal pattern, wherein the image processor is configured to generate the temporal pattern with a variation rate, it is of advantage if the output data rate is larger than the variation rate. Thus, the temporal variation of the pattern can be smoothly displayed. The output data rate usually corresponds to a frame rate, and thus should be higher than the flicker fusion rate in order to produce a smooth sequence of images. In contrast, the variation rate is configured to produce a perceptible motion.

The pseudo-color image data may also comprise symbols, such as arrows, letters and numerals.

The input interface may, according to another aspect of the invention, comprise an input section configured to receive at least one of ultrasound data and fluorescence image data as auxiliary input data. The ultrasound data may be a one-dimensional data field as generated by the recordings of an ultrasound microphone. The ultrasound data may in addition or alternatively be multi-dimensional such as produced by an ultrasound sensor head or scanner. In particular, the ultrasound data may be two-dimensional or three-dimensional (in spatial dimensions) and contain, at each data point representing a spatial location additional data, such as a velocity and direction of flow or movement.

The image processor may comprise spatial alignment module, which is adapted to spatially align the input image data and any auxiliary input image data so that spatial features in both data are spatially congruent.

If for example the input image data are image data received from a visible-light camera and the auxiliary input data are fluorescent-light image data received from a fluorescence-camera, where both the visible-light camera and the fluorescence camera have overlapping field of views, the spatial alignment module rectifies at least one of the images so that the spatial features are of the same size and orientation in both image data. This allows exact merging of the various image data in the output image data. The auxiliary input data in such a case may alternatively or additionally comprise data from sensors detecting other physical, preferably non-visible, quantities. These data may be rectified in the same way using the spatial alignment module as the fluorescence image data mentioned above.

If the fluorescence takes place in the NIR range, the fluorescence camera may be a NIR camera and the fluorescence image data may be NIR image data.

If the auxiliary data are image data from a fluorescence camera, in particular fluorescent-light image data, the temporal and/or spatial pattern may depend on the saturation and/or brightness of the fluorescence image data. The pseudo-color pattern field may further be generated where two different colors or wavelengths in the fluorescence-image data overlap and, as an optional further criterion, each color or wavelength exceeds a minimum intensity.

If the auxiliary input image data comprise a three-dimensional data set, the temporal and/or spatial pattern may depend on the location, in particular depth, at which the pre-determined condition for switching this pattern on is detected. This allows including data in the output image data which represent structural and/or physical features in a plane that is below what is represented in the input image data, such as a blood vessel or a bone beneath a tumor marked by a fluorophore.

According to another aspect, the image processor may be configured to generate subsequent sets of pseudo-color image data according to a temporal modulation scheme. The temporal modulation scheme is preferably stored in the image processor. The modulation scheme may comprise at least one of a time-varying alteration between sets of different pseudo-color image data representing different pseudo-colors, pseudo-color image data of different saturation and/or brightness, different pseudo-color image data representing cyclically changing patterns.

The modulation scheme may further comprise a propagating wave pattern, wherein, in the propagating wave pattern, at least one of speed, direction and wavelength depends on the content of the auxiliary input data.

The aspect of the output image data comprising three-dimensional image data and of the image processor being configured to generate and/or to process three-dimensional output image data, in which pseudo-color image data are generated at different depths or planes of the three-dimensional image data depending on the content of the auxiliary data may be realized independent of the generation of a pseudo-color pattern field.

Three-dimensional input image data may be generated by Z-stacking using either a visible-light camera or a fluorescence camera. The pseudo-color image data may thus be used to present three-dimensional features. The three-dimensionality of the image data may, as has already been explained above, also result from a three-dimensional ultrasound image.

The output image data may be, according to a further aspect of the invention, two-dimensional image data and at least one of the input image data and auxiliary input data may comprise three-dimensional data. The image processor is adapted to compute the two-dimensional output image data from the three-dimensional input data using pseudo-colors for features which are not in the plane of the two-dimensional output image data. The pseudo-color image data, in particular a pseudo-color pattern field thereof, may be used to display data content of a part of the three-dimensional input image and/or the three-dimensional auxiliary input data that is not within the plane rendered in the two-dimensional output data.

The use of a pseudo-color image in connection with three-dimensional input image or auxiliary input data is advantageous on its own, i.e. without using pseudo-color pattern fields.

The medical observation device may comprise at least one visible-light camera which is connected to the input interface for providing the input image data. The visible-light camera may provide three-dimensional input image data or two-dimensional input image data. The medical observation device may further comprise at least one sensor device configured to sense a physical quantity which is not electromagnetic radiation in the visible wavelength range, such as at least one fluorescence camera, at least one ultrasound sensor, at least one microradiation camera, at least one gamma ray camera, at least one Roentgen camera and/or at least one thermographic camera. These devices may be connected to the input interface for providing the auxiliary input data. The auxiliary input data may be one-dimensional, two-dimensional, three-dimensional or provide sets of auxiliary input data of different dimensionalities.

The medical observation device is preferably configured to discern, process and display more than one fluorophore, i.e. more than one different fluorescent spectra. For this, at least one of a fluorescence camera, a light source and a visible-light camera may be provided with a filter arrangement for selectively blocking and/or transmitting the fluorescent spectra. The pseudo-color image comprises in such a case preferably at least two different pseudo-colors, wherein each pseudo-color is assigned to a different fluorescence spectrum, i.e. to a different fluorophore.

The image processor and its constituents may be hardware devices or may be, at least partly, represented by software modules executed in a multi-purpose computer or processor.

The invention may employ the merging of the pseudo-color image data with the input image data as described in the parallel application EP 16 155 625.3.

In the following, the invention is described exemplarily with reference to the accompanying figures. In the figures, elements which correspond to each other with respect to at least one of function and design are assigned identical reference numerals.

The combination of features shown in the figures and described below is only an example. Individual features can be added or omitted if the technical effect of that particular feature is described above is needed or not necessary for a particular application.

In the figures,
- Figure 1: shows schematically an embodiment of a medical observation device according to the invention;
- Figure 2: shows a schematic representation of a method of operation for superposing medical images according to the invention;
- Figure 3: shows schematically an example of a set of input image data and auxiliary input data being merged to output image data;
- Figure 4: shows schematically another example of input image data and auxiliary input data being merged to an output image;
- Figure 5: shows schematically pseudo-color pattern fields having spatial modulation within pseudo-color image data;
- Figure 6A-6C: show schematically a time-modulated pattern;
- Figure 7A-7I: show schematically a time-modulated pattern.

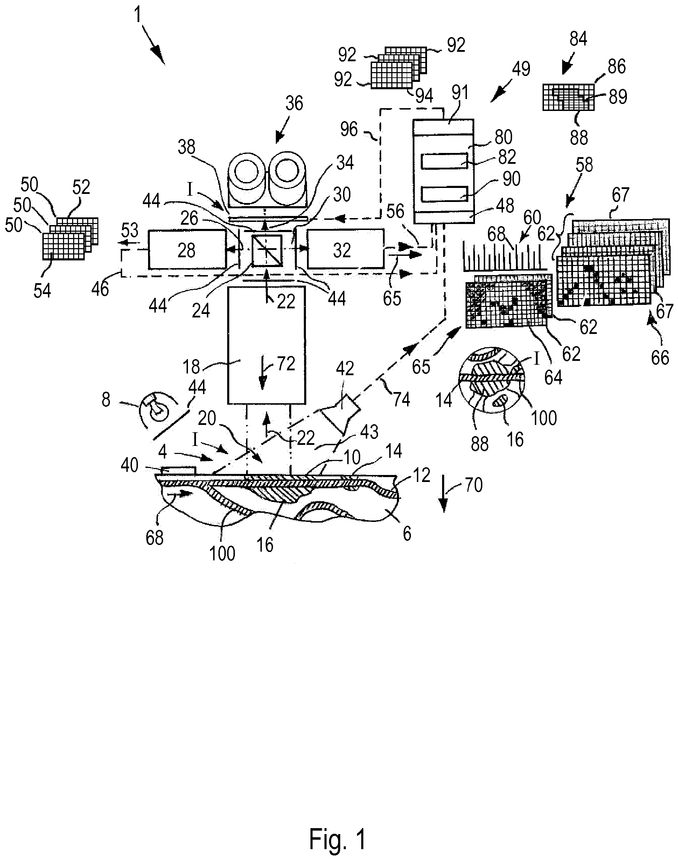

First, the structure of a medical observation device 1 which includes the invention is described with reference to Figure 1.

Just by way of example, the medical observation device 1 is shown in Figure 1 to be a microscope. The medical observation device 1 may also be an endoscope.

The medical observation device 1 is used to inspect an object 4, such as live tissue 6, for diagnostic or surgery purposes. In the object 4, one or more fluorophores may be present.

Upon excitation by certain wavelengths of a light source 8, the fluorophores emit fluorescence light at different peak emission wavelengths, e.g. in the NIR range. If two or more fluorophores are used, they may emit light of different peak emission wavelengths, or just have different spectra. Different fluorophores may be used to mark different types 10, 12 of live tissue 6, so that these types 10, 12 may be discerned by the different fluorescent wavelengths they emit. The light source 8 may also serve as illumination for observation of the object in the visible-light range.

For example, a first fluorophore 14, which is schematically represented in Figure 1 by one type of hatching, may be used for marking specifically one type 12 of live tissue 6, for example blood vessels. A second fluorophore 16, represented in Figure 1 by another type of hatching, may e.g. mark exclusively another type 10 of live tissue 6, for example tumor cells.

The medical observation device 1 may comprise an optical system 18, such as a zoom magnifying lens. In the case of an endoscope, the optical system 18 may also comprise fiber optics. The optical system 18 is, in operation of the medical observation device 1 and its medical image superposition device 49, directed onto at least a section of the live tissue 6. The visible section of the object 4 is determined by the field of view 20 of the optical system 18. Light reflected off the live tissue 6 together with any fluorescent light emitted from the at least one fluorophore 14, 16 is collected by the optical system 8 and directed to one or more beam splitter system 24 which contains at least one beam splitter.

A part 26 of the light 22 is directed to a visible-light camera 28 by the at least one beam splitter system 24. Another part 30 of the light 22 may be directed to a fluorescence camera 32. Another part 34 of the light 22 may be directed to an ocular 36 which may be monocular or binocular.

The ocular 36 may be directed onto a transmissive display 38, which is arranged between the ocular 36 and the at least one beam splitter system 24. The transmissive display 38 allows the part 34 of the light 22 to pass and to superpose a picture, which is currently displayed on the display 38, onto the image provided by the optical system 18.

Alternatively, the display 38 may not be transmissive. In this case, the display 38 may render a live view of the object 4 with additional information or display any other information that an operator of the medical image superposition device 49 or the medical observation device 1 requests. In particular, the background image, which in the case of a transmissive display 38 would be provided directly by the optical system 18, may be instead provided in real time by the visible-light camera 28.

The medical observation device 1 may further comprise at least one sensor 40, e.g. an ultrasound or blood-flow sensor, which may be in contact with the object 4.

In addition to the at least one sensor 40 at east one further sensor device 42 may be provided. The sensor device 42 is preferably a non-optical sensing device that is configured to sense non-visible physical quantities such as microradio, gamma, Roentgen, infrared, or sound data. The non-optical sensing device 42 provides two- or three-dimensional data. Examples for the non-optical sensing device 42 are microradiography cameras, ultrasound sensor heads, thermographic cameras, gamma ray cameras and Roentgen cameras.

The non-optical sensing device preferably has a sensor field 43, which overlaps the field of view 20 so that the data captured by the optical system 28 and the non-optical sensing device 42 represent different physical quantities from the same area of the object 4.

In order to separate the light in the visible-light range from the light in the fluorescence wavelengths and in order to avoid reflections, a filter arrangement 44 may be used preferably immediately in front of at least one of the light source 8, the beam splitter system 24, the visible-light camera 28, the fluorescence camera 32, the ocular 36 and the display 38.

The visible-light camera 28 is connected via a data connection 46 to an input interface 48 of a medical image superposition device 49. The medical superposition device may be realized by hardware dedicated to carry out specific functions, such as an ASIC, by software executed by a general-purpose computing device, or a combination of both.

The input interface 48 receives, in operation of the medical image superposition device 49 and the medical observation device 1 subsequent sets 50 of input image data 52. The input image data 52 is two-dimensional and organized to contain pixels 54. In the embodiment of Fig. 1, the subsequent sets 50 of the input image data 52 correspond to the sequence of visible-light image data 53 provided by the camera 28.

The input interface 48 is further configured to receive auxiliary input data 58, which may be one-dimensional auxiliary input data 60, sets 62 of two-dimensional auxiliary input data 64 or three-dimensional auxiliary input data 66 consisting of several planes 67 of two-dimensional data. Of course, the auxiliary input date 58 may also comprise data of a higher dimensionality, e.g. if hyperspectral cameras are used or ultrasound sensor heads, which output three-dimensional pictures, which contain additional physical data such as blood flow velocity and direction for each pixel or voxel.

The fluorescence camera 32 may transmit, via the data connection 56, subsequent sets 62 of two-dimensionally auxiliary input data 64 which may in particular represent fluorescence image data 65 from the fluorescence camera 32. The fluorescence image data 65 may have the same format as the input image data 52 but at a different resolution.

The sensor 40 may provide one-dimensional auxiliary input data e.g. in the form of a time sequence blood flow velocity 68 in the blood vessel 12.

The sensing device 42 may provide subsequent sets 62 of two- or three-dimensional auxiliary input data 64 e.g. in planes 67 parallel to the depth direction 70 of the object 4, i.e. to the viewing direction 72 of the medical observation device 1.

The sensing device 42 may be connected to the input interface 48 of the medical superposition device 1 by a data connection 74. Another data connection, not shown for simplicity's sake, may exist between the ultrasound sensor 42 and the input interface 48. All data connections may comprise wired and/or wireless sections.

It is to be understood that auxiliary input data 58 can be received by the medical image superposition device 49 from any one of the described devices, e.g. only from the fluorescence camera 38 or only from the ultrasound sensor 40, as well as any combination of such devices. Moreover, other devices may also be employed to provide auxiliary input data 58, although not shown in Figure 1. For example, a thermographic camera, a microradiography camera, a Roentgen camera and/or a gamma ray camera may be used instead of or in addition to an ultrasound sensor and/or the fluorescence camera.

The medical image superposition device 49 comprises an image processor 80 which is configured to blend the input image data 52 from the visible-light camera 28 with the auxiliary input data 58 and provide output image data 81 which contain both at least parts of the input image data 52 and at least parts of the auxiliary input data 58. The input image data 52 are used as background data whereas the auxiliary input data 58 are assigned at least one pseudo-color and/or a pseudo-color pattern before they are merged with the input image data 52. The pseudo-color is manually or automatically by the image processor 80 selected from a list of colors which is not present in the image input date 58 and which preferably does not exist in tissue 6, such as neon colors. Preferably, each different physical quantity is assigned a different pseudo-color and/or pseudo-color pattern.

For this, the image processor 80 comprises a pseudo-color image generator 82. The pseudocolor image generator 82 assigns a pseudo-color to the auxiliary input data 58 depending on the content and/or source of the auxiliary input image data 58. Preferably for each set 50 of image data 52, the pseudo-color image generator 82 is configured to generate a corresponding set 84 of pseudo-color image data 86 in real time. The pseudo-color image data 86 are then blended with the input image data 52.

The image processor 80, in particular the pseudo-color image generator 82 is further configured to generate at least one pseudo-color pattern 87 within the pseudo-color image data 86. The pattern 87 extends over a coherent section of the pseudo-color image data 86 and thus forms a pseudo-color pattern field 88. The pattern 87 may comprise a temporal modulation and/or a spatial modulation of zones of pseudo-color within the pseudo-color image data 65. A spatially modulated pattern has regularity in space, a temporally modulated pattern has regularity over time. Regularity means that the pattern repeats in a predictable way.

If the pattern 87 has temporal modification, it is generated by the pseudo-color image generator 82 to have a variation rate, which represents the time between successive repetitions of the pattern 87.

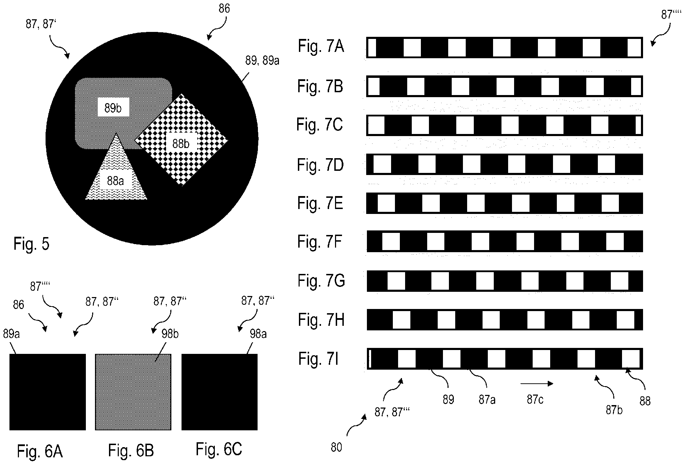

The pattern 87 is generated in dependence of the content of the auxiliary input data 58. For example, a pattern 87 may be generated instead of a solid pseudo-color by the pseudo-color image generator 82 in an area of the auxiliary input data 58, in particular the fluorescent-light image data 65, where a threshold, for example a brightness threshold, is not met. Different patterns 87 may be generated depending on how far the content is below the threshold. This is exemplarily described in the following with reference to Figures 5, 6A to 6C and 7A to 7I. Fig. 5 shows a pseudo-color pattern 87' having spatial modulation, Fig. 6A to 6C show a pattern 87" having temporal modulation, and Fig. 7A to 7I show a pattern 87‴ having both temporal and spatial modulation, the temporal modulation being based on a temporal modulation scheme 87ʺʺ.

In Fig. 5, a schematic representation of one set of pseudo-color image data 86 is given. The pseudo-color image data 86 contains at least one pseudo-color 89, in this example two different pseudo-colors 89a and 89b, and two different pseudo-color pattern fields 88 containing different pseudo-color patterns 87, having in this example only spatial modulation. One pseudo-color pattern field 88a for examples is built up of a repeating wave-form template and can be used to designate an area in the auxiliary input data 58, e.g. from an ultrasound sensor, in which an increased liquid content has been discovered by automatically comparing the auxiliary input data 58 with a data library. Another pseudo-color pattem field 88b can be used to designate an area of the auxiliary input data 58, in which the intensity is below or above a threshold. As the patterns 87 have only spatial modulation, they do not change over time. The extent of the pseudocolor pattern field 88, however may change over time, as it depends on the content of the input image data 52 and/or the auxiliary input data 58.

Fig. 6A to 6C show a pseudo-color pattern 87 with temporal modification. In this example, the temporal modification is a simple blinking operation over time. In Fig. 6A, the pseudo-color pattern field 88 contains a first pseudo-color 89a. In Fig. 6B, the same pseudo-color pattern field 88 is shown to contain a second pseudo-color 89b (or no pseudo-color) at a later point of time, which has replaced the first pseudo-color 89a. At again a later point in time, shown in Fig. 7C, the pseudo-color pattern field 88 is restored to the state shown in Fig. 6A. Thus, a repetitive pattern is displayed over time. The time-period between successive repetitions is determined by the variation rate of the temporally modulated pseudo-color pattern 87. The variation rate is determined in the pseudo-color image generator 82 depending on the content of the input image data 52 and/or the auxiliary input data 58. In order to have a clearly visible temporal modulation, the variation rate is smaller than the flicker fusion rate, in particular less than half the flicker fusion rate.

The temporal modulation shown in Fig. 6A to 6C may for example be assigned to auxiliary input data, in which the intensity is above a threshold, and which if assigned to a static pseudo-color, would, not be discernible in the pseudo-color image data 86, e.g. because saturation has been reached. In such a case, instead of showing a second pseudo-color 98b, the pseudo-color can be switched of in the pseudo-color image data 86.

Another example, where the temporal modulation shown in Fig. 6A to 6C may be used is an area, where otherwise two static pseudo-colors would have been assigned. Using the temporal modification, it can be clearly indicated that the pseudo-color pattern field 88 results from auxiliary input data of which the content satisfies the criteria for the assignment of more than one pseudo-color 89. Of course, the temporal modulation shown in Fig. 6A to 6C can be extended to contain more than two pseudo-colors 89a, 89b in sequence.

The pseudo-color pattern field 88 may comprise a pseudo-color pattern field 88 which has both temporal and spatial modulation. This is shown in Fig. 7A to 7I, which depict a pattern 87 comprising at least one solid pseudo-color pattern area 87a which assumes a different location in subsequent sets 84 of pseudo-color image data 86 to produce a progressing wave pattern 87b progressing in the direction of the arrow 87c. Each of the Fig. 7A to 7I shows one of the subsequent sets 84 during one variation period. At the end of the variation period, in Fig. 7I, the cycle is repeated by starting with the pattern of Fig. 7A.

The pseudo-color pattern field 88 shows regularity in its change both over time and space in that the at least one solid pseudo-color pattern area 87a alternates regularly in space with a transparent area or an area filled with another pseudo-color, and in that the same spatial pattern is contained in the pseudo-color pattern field 88 after each passing of the variation rate. It is to note, that the variation rate itself may change over time depending on the content of the auxiliary input data 58.

The pseudo-color pattern 87 shown in Fig. 7A to 7I may e.g. used for auxiliary input data 58 which represent a velocity, flow rate and/or direction, such as blood flow data. The velocity represented in the auxiliary input data 59 may be represented by the variation rate, the flow rate by the spatial variation rate or the intensity of the pseudo-color 89 in the pattern 87. The extent of the pseudo-color pattern field 88 is determined automatically from the auxiliary input data 58 for each set 52.

The image processor 80 may further comprise a spatial alignment module 90. The spatial alignment module 90 is configured to rectify the input image data 52 and the auxiliary input data 58 spatially so that each set 52, 62 is spatially congruent to each other. This can for example be done by algorithmically correlating spatial features which are present both in the input image data 52 and the auxiliary input image data 58. The spatial alignment module 90 is configured to rectify at least one of the image input data 52 and the spatial input data 58 by morphing the image so that correlating structures are aligned. The morphing operation may comprise stretching, rotating, adjusting resolution, and/or warping of at least one of the input image data and the auxiliary input data.

For example, fluorescence image data from the fluorescence camera 32 may be slightly rotated, warped and displaced relative to the visible-light image data from the visible-light camera 82. Further, two-dimensional data 64 from the ultrasound sensor 40 may have a different scale and resolution, and be warped and rotated with respect to the input image data 50 and/or the fluorescent-light image data 65 from the fluorescence camera. In order to correctly superpose these data, the spatial alignment module 90 is configured to execute a correlation algorithm to identify common structures in the respective data 52, 58 and compute their respective orientation and scale. The spatial rectification is performed depending on the result of this algorithm for each synchronized set of input image data and auxiliary input data.

Further, the spatial alignment module 90 may comprise transfer functions of the devices which generate the auxiliary input data 58, which transfer functions have been obtained by initial calibration processes. The transfer functions may be used to correct errors in the optical system such as vignetting, distortion and/or aberration.

The medical image superposition device 49 further comprises an output interface 91, which is configured to output subsequent sets 92 of output image data 94. The output image data 94 result from the merger of the pseudo-color image data 86 and the input image data 50.

An example for such a merger is given in applicant's application EP 16 155625.3. Although the merging is described in this reference in relation to fluorescent-light image data only, the merging can be used for any other type of two-dimensional auxiliary input data 58, 64, 67 without any further change. S

The output image data 94 are output at an output data rate which is preferably larger than the highest variation rate of a temporarily modulated pattern 87 in the output image data 94 by an order of magnitude. In particular, the output data rate is higher than the flicker fusion rate, in particular higher than 25 Hz. The variation rate in contrast is smaller than the flicker fusion rate, so that the regular variation of the pseudo-color pattern 87 is clearly visible.

It is to be understood, that recognition of the pattern 87 does not depend on cognitive processes of the human mind but that the pattern 87 can be recognized by any automated pattern recognition process due to its regularity in at least one of the spatial and temporal domain.

The display 38 is connected via a data connection 96 to the output interface 91. In the display 38, the output image data 94 are displayed. If the display 38 is transmittive, the input image data 52 may be omitted from the output image data 92, and only the pseudo-color image data 86 may be displayed. The input image data 50 in this case are used only for aligning the pseudo-color image data 86 and the pattern 87 with the input image data 52. As the input image data 50 give an accurate rendition of what is seen through the display 38, the effect is the same as using a non-transmittive display and displaying the merged input image data 52 and the pseudo-color image 86.

A schematic example of what is seen through the ocular 36 is presented in detail I of Figure 1.

The fluorophores 14, 16 are each assigned a different pseudo-color by the pseudo-color image generator 82. The output image data 94 comprise at least one pseudo-color pattern field 88, which is generated in real time by the pseudo-color image generator 82 and which has a temporal and/or spatial modulation. The pattern field 88 comprises the pseudo-color pattern 87 and is generated depending on the content of the input image data 52 and/or the auxiliary input data 58.

For example if, in the fluorescent-light image data 65 of the fluorescence camera 32, there are areas within the fluorescent-light image data 65 having a fluorescence intensity below a predetermined but preferably adjustable threshold, they may be marked with a pseudo-color pattern 87 in which the pseudo-color 89 may have at least half of their full brightness, so that in spite of the low intensity in the fluorescent-light image data 65, this area is still visible to an observer. Alternatively, or additionally, a different pattern 87 may be used in an area where the fluorescent-light image data have an intensity which exceeds a predetermined but preferably adjustable threshold. This may indicate to an observer that these data are unreliable or that he has to adjust the camera sensitivity.

The medical image superposition device 49 and the medical observation device 1 may provide two- and/or three-dimensional images in the display 38 and, accordingly, the display 38 may be a 2D- or 3D-display.

If auxiliary input data 58 are used which comprise data from locations which are situated in the depth direction 70 beneath the plane of the field of view 20 of the optical system 18 and thus are not contained in the visible-light image data 53 from the visible-light camera 28 or in the fluorescent-light image data 65 from the fluorescence camera 32, they may be added to the pseudo-color image data 86 using a pseudo-color pattern 87. For example, if the ultrasound sensor 40 or 42 has detected a coherent structure 100 in the object 4, such as a large blood vessel or a nerve beneath the field of view 20, the structure 100 may be rendered by using a pseudo-color pattern field 88 in the pseudo-color image data and ultimately in the output image data.

Various auxiliary input data 58 from different devices may be simultaneously displayed by using different pseudo-colors and different patterns 87.

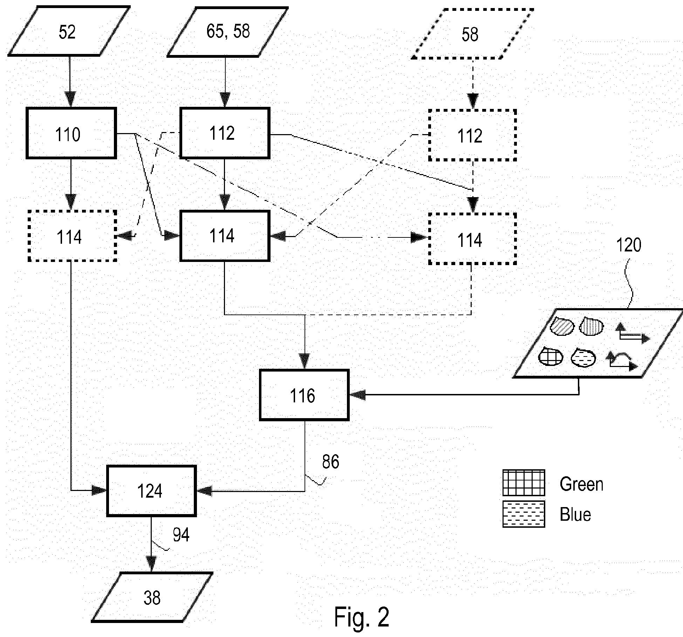

Next, the process of generating output image data from the input image data and auxiliary input data via pseudo-color image data is explained with reference to Figure 2. In Fig. 2 optional steps are indicated by dashed lines.

Figure 2 shows that the input image data 52 are pre-processed, in a first step 110, which may be carried out by the medical image superposing device 1. The pre-processing step 110 may include any one or any combination of histogram equalization, automatic adjustment of contrasts, compensation of aberration, vignetting and distortion errors caused by the optical system 18 as well as noise reduction, but may not be limited to these.

The auxiliary input data 58, such as the fluorescent-light image data 65 or other and further auxiliary input data 58, such as ultrasound data, microradiography image data, thermographic image data and/or Roentgen image data may also undergo a pre-processing step 112 to compensate at least one error, noise and distortions. The pre-processing algorithms carried out in step 112 may in general be the same as for the input image data 52 in step 110, using however, different parameters to account for the different physical parameters and systems. For example, the compensation of errors introduced by the optical system 18 may be different for the fluorescent-light image data 63 than for the visible-light image data 53.

In particular for the auxiliary input data 58, the pre-processing 112 may also comprise a threshold comparison, in which those auxiliary input data with a content below a certain sensitivity threshold are for example blanked or deleted so that they will not be contained in the output image data. For example, pixels may be blanked or set to zero or set to transparent in auxiliary input data 58, if the intensity of that pixel is below a sensitivity threshold. The sensitivity threshold may be determined by a user of the medical image superposing device 1 or medical observation device 1. Thus, pixels which represent only a very low signal strength, i.e. fluorescence level or ultrasound reflectivity, may thus not be considered in the pseudo-color image generation.

The next step 114 comprises spatial adjustment of at least one of the input image data 52 and the auxiliary input data 58, in particular of two-dimensional and/or three-dimensional auxiliary input data so that spatial features are located at the same location and in the same size and orientation in both the input image data 52 and the auxiliary input data 58. At the end of spatial adjustment 114, the input image data and the auxiliary input data are at least substantially spatially congruent. Preferably, the input image data 52 are used for reference in the spatial alignment step 114. The auxiliary input data 58 are thus modified in the spatial alignment step 114, whereas the input image data 53 are left unchanged. This is of particular advantage if the auxiliary input data 58 contain less data than the input image data, e.g. because pixels have been blanked and/or because the resolution measured in pixel of the fluorescence camera 32 and/or a microradiographic, thermographic or gamma-ray camera and/or ultrasound sensor is smaller than the resolution of the visible-light camera 28. However, leaving the input image data unchanged will maintain the field of view 20 of the optical system 18 and the visible-light camera 28 and thus will render faithfully the filed of view 20 on the display 38.

In the spatial alignment step 114 the auxiliary input data 58 of a set 62 or a plane 67 may be distorted, rotated and changed in its resolution to spatially correlate it to the input image data 52. The input image data 52 and the auxiliary input data 58 may be time-stamped so that a set of input image data 52 is processed together with a set of auxiliary input data 58 that has been sampled at the same time as the input image data.

Next, in step 116, pseudo-color image data are generated from the auxiliary input data 58.

For example, the different fluorescence colors in a fluorescent-light image data 63, which are in the NIR range and thus invisible to humans, may be assigned different pseudo-colors in the visible range. Further, auxiliary input data 58 representing invisible physical quantities, i.e. physical quantities not being electromagnetic waves in the visible light range, such as ultrasound, microradiation, gamma-ray or thermographic sensing, may also be assigned pseudo-colors. Preferably, each sensing modality is assigned a different pseudo-color and/or a different pattern.

Using many different types of auxiliary input data 58 in a single set of output image data may result in the use of too many pseudo-colors. Thus, a pattern using the same pseudo-color may be used to keep the numbers of different pseudo-colors small. Further, use of a pattern 87 such as a hatching only obscures part of the underlying input image data, and allows having a better view of the underlying input image data.

Further, if the content of the auxiliary input data 58 designates a low intensity of the recorded signal in the auxiliary input data 58, they would be visualized with only subtle pseudo-coloring. Such a subtle pseudo-coloring may be insufficiently set apart from non-pseudo-colored areas. Thus, these areas should be not filled with a solid pseudo-color but with a pattern in which the pseudo-color has a high brightness, e.g. of at least 50 % of the maximum brightness value.

The different patterns and pseudo-colors are preferably selected automatically from a storage 120 maintained in the medical image superposing device 2. The user may be able to preset certain assignments, i.e. to assign e.g. a particular pseudo-color and/or pattern to specific data sources, to specific content of data from a data source, such as to specific fluorophores, to pixel intensities, or to alteration patterns of data, such as rates of change.

In step 116, subsequent sets 84 of pseudo-color image data 86 are obtained, in which at least one pseudo-color pattern field 88 is filled with a pattern 87 depending on the content of at least one of the input image data 52 and the auxiliary input data 58.

In the next step 124, the pseudo-color image and the input image are merged. It is to be understood that this merging occurs for each data set in the subsequent sets of input image data 52 and auxiliary input data 58 to allow a real-time processing.

The merging process is described in detail in the parallel application EP 16 155625.3. As a result of the image merging step 124, the output image data 94 are obtained and finally displayed on the display 38.

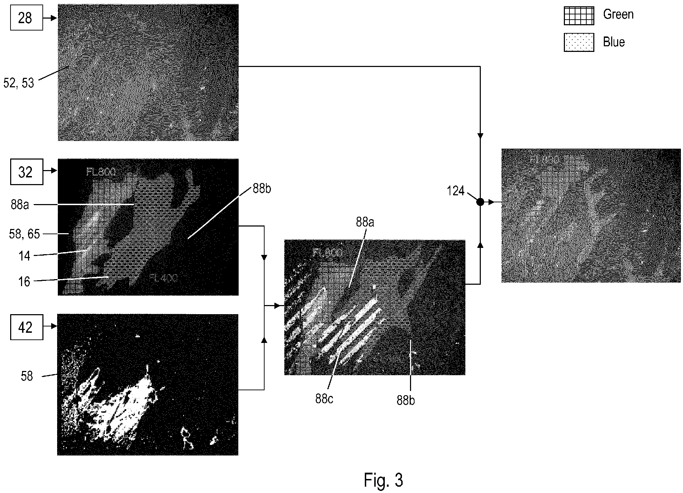

In Figure 3, the process depicted in Figure 2 is further explained.

Image input data 52 represent visible-light image data 53 from the visible-light camera 28. Fluorescent-light image data 63 are obtained as auxiliary input data 58 from an fluorescence camera 32 and contain the emission spectra of at least two fluorophores 14, 16 which are assigned different pseudo-colors designated FL800 and FL400 in Figure 3.

At least one pseudo-color pattern field 88 has been generated in the pseudo-color image data 86 depending on the content of the fluorescence image data 63. The pseudo-color pattern field 88a designates an area in which the two fluorophores 14, 16 overlap. The pseudo-color pattern field 88a may contain one of or both the pseudo-colors which have been assigned to the respective fluorophores 14, 16 or the corresponding emission spectra, or a different pseudo-color. Another synthetically generated pseudo-color pattern field 88c designates an area in which one of the pseudo-colors has a very low intensity. This pseudo-color pattern field 98 may use the same pseudo-color assigned to the respective fluorophore or fluorescence emission spectrum.

Two-dimensional auxiliary input data 58 from the ultrasound sensor 42 are also transformed to pseudo-color image data 86. A pattern field 88 is synthetically generated in areas where the pixel intensity in the auxiliary input data 58 from the ultrasound is above a threshold. The pseudo-color images are then merged with the image input data 52 to obtain output image data 94 containing at least one pseudo-color pattern field 88.

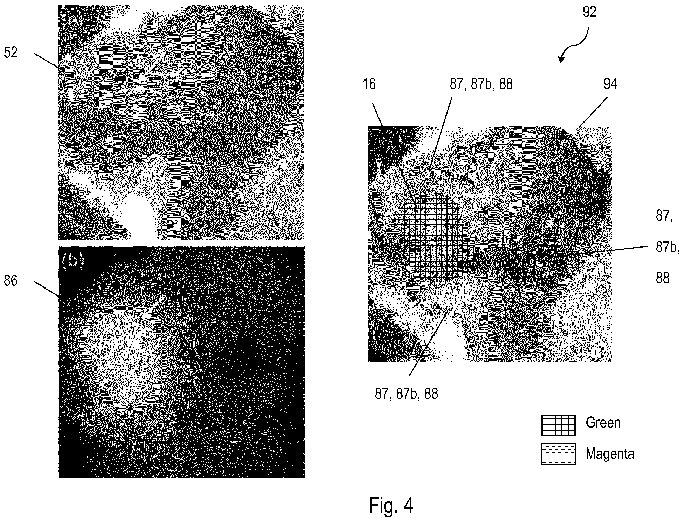

In Figure 4, a set 92 of output image data 94 has been generated by merging fluorescent-light image data 65 containing a fluorescent light from a tumor-marking fluorophore 16 with ultrasound image data and visible-light image data 53.

The at least one pseudo-color pattern field 88 in the output image data 94 may have a temporal modulation as indicated by arrow 134. The area occupied by the pseudo-color pattern field 88 corresponds to areas which were automatically recognized as blood vessels having a blood flow velocity and direction in each subsequent set. The pseudo-color pattern field 88 is a wave pattern which is modified in subsequent sets of output image data 94 to produce a wave pattern 87b which propagates in the direction of arrow 134.

The speed and direction of the wave propagation 34 depends on the blood flow direction and the blood flow speed as contained in the auxiliary input data 58 from the ultrasound sensor 40 or 42. The wave length may be selected to be dependent on the distance of the blood vessel from the surface. Information on the location and extent of the blood vessel and the speed and direction of blood flow can be extracted from the ultrasound data by algorithms executed by the image processor 80.

### Reference Numerals

- 1: medical observation device
- 4: object
- 6: live tissue
- 8: light source
- 10: type of tissue, timorous tissue
- 12: type of tissue, blood vessel
- 14: first fluorophore
- 16: second fluorophore
- 18: optical system
- 20: field of view
- 22: light collected by optical system
- 24: beam splitter system
- 26: light branched off to visible-light camera
- 28: visible-light camera
- 30: light branched-off to fluorescence camera
- 32: fluorescence camera
- 34: light branched-off to ocular or binocular
- 36: ocular
- 38: display
- 40: ultrasound sensor
- 42: non-optical sensing device
- 43: field of view of non-optical sensing device
- 44: optical filter arrangement
- 46: data connection
- 48: input interface
- 49: medical image superposition device
- 50: sets of input image data
- 52: input image data
- 53: visible-light image data
- 54: pixel
- 56: data connection
- 58: auxiliary input data
- 60: one-dimensional auxiliary input data
- 62: set of two-dimensional auxiliary input data
- 64: two-dimensional auxiliary input data
- 65: fluorescent-light image data
- 66: three-dimensional auxiliary input data
- 67: plane of three-dimensional input data
- 68: blood flow velocity / direction
- 70: depth direction
- 72: viewing direction
- 74: data connection
- 80: image processor
- 82: pseudo-color image generator
- 81: output image data
- 84: set of pseudo-color image data
- 86: pseudo-color image data
- 87: pseudo-color pattern
- 87': pseudo-color pattern having spatial modulation
- 87": pseudo-color pattern having temporal modulation
- 87‴: pseudo-color pattern having temporal and spatial modulation
- 87ʺʺ: temporal modulation scheme
- 87a: solid pseudo-color area within pseudo-color pattern
- 87b: progressive-wave pattern
- 87c: direction of progression
- 88: pseudo-color pattern field
- 88a: pseudo-color pattern field indicating data overlap
- 88b: pseudo-color pattern field indicating low intensity
- 89: pseudo-color
- 89a: one pseudo-color
- 89b: different pseudo-color
- 90: spatial image alignment module
- 91: output interface
- 92: set of output image data,
- 94: output image data
- 96: data connection
- 98: pseudo-color pattern field
- 100: large blood vessel
- 110: step of pre-processing input image data
- 112: step of pre-processing auxiliary input data
- 114: spatial image alignment
- 116: pseudo-color image generation
- 120: pseudo-color and/or pseudo-color pattern storage
- 124: image merging
- 134: arrow

## Claims

1. Microscope, comprising an input interface (48) and an output interface (90),
the input interface (48) being configured to receive subsequent sets (50) of input image data (52) and auxiliary input data (58),
the output interface (91) being configured to output subsequent sets (92) of output image data (94) at an output data rate,
the microscope further comprising an image processor (80),
wherein the image processor is connected to the input interface (48) for receiving the subsequent sets (50) of the input image data (52) and the auxiliary input data (58), and to the output interface (91) for outputting the subsequent sets (92) of the output image data (94),
the image processor further being configured to generate, in real time, subsequent sets (84) of pseudo-color image data (86) depending on a content of at least one of the auxiliary input data (58) and the input image data (52),
wherein the output image data (94) are three-dimensional and
wherein the image processor (80) is configured to generate the pseudo-color image data (86) at different depth layers of the three-dimensional output image data depending on the content of the auxiliary input data (58),
wherein the image processor (80) is further configured to generate a synthetic pseudo-color pattern field (88) within the pseudo-color image data (86) depending on the content of at least one of the input image data (52), the auxiliary input data (58) and the pseudo-color image data (86), the pseudo-color pattern field (88) comprising a temporally modulated pattern (87", 87‴) having a variation rate, and
wherein the image processor is configured to compute the variation rate of the temporally modulated pattern (87", 87‴) depending on the content of at least one of the input image data (52) and the auxiliary input data (58), wherein the image processor (80) is configured to merge the pseudo-color image data (86) and at least one section of the input image data (52) to generate the output image data (94) containing the pseudo-color pattern field (88).

2. Microscope according to claim 1, wherein the pseudo-color pattern field (88) further comprises a spatially changing pseudo-color pattern (87, 87').

3. Microscope according to claim 2, wherein subsequent sets (50) of the output image data (52) comprise the pseudo-color pattern field (88), and wherein the temporally modulated pattern comprises a propagating wave pattern (87b) of which at least one of a speed, direction and wavelength depends on the content of the auxiliary input data (58).

4. Microscope according to any one of claims 1 to 3, wherein the output image data (52) contains the pseudo-color pattern field (88) in an area where the contents of the auxiliary input data (58) are at least one of below and above a threshold.

5. Microscope according to any one of claims 1 to 4, wherein the auxiliary input data (58) comprise three-dimensional auxiliary input data (66) and wherein the image processor (80) is adapted to generate the pseudo-color pattern field (88) depending on the content of the three-dimensional auxiliary input data (67).

6. Microscope according to any one of claims 1 to 5, comprising a visible-light camera (28) connected to the input interface (48) for providing the subsequent sets (50) of the input image data (52) and comprising at least one of an fluorescence camera (32) and an ultrasound sensor (40, 42) connected to the input interface (48) for providing the auxiliary input data (58).

7. Microscope according to claim 6, comprising the fluorescence camera (32) and a light source (8), at least one of the fluorescence camera (32), the light source (8) and the visible-light camera (28) being provided with a filter arrangement (44) for at least two different fluorophores (14, 16), each fluorophore (14, 16) having different peak emission wavelengths, the output image data (94) comprising at least two pseudo-colors (89) and the image processor (80) being configured to assign a different pseudo-color (89) being to each of the different fluorescence peak emission wavelengths.

8. Microscope according to claim 6 or 7, further comprising the ultrasound sensor (40, 42), wherein the image processor (80) is configured according to any one of claims 2 to 6 and further configured to generate the pseudo-color pattern field (88) in the output image data (94) depending on the content of the auxiliary input data (58) from the ultrasound sensor (40, 42).

9. Microscope according to any one of claims 1 to 8, comprising an optical system (18), a transmissive display (38), an ocular (36), and at least one beam splitter system (24), the transmissive display (38) being arranged between the ocular (36) and the at least one beam splitter system (24), the ocular (36) being directed onto the transmissive display (38), the transmissive display (38) being configured to allow a part (34) of light (22) from an inspected object (4) to pass and to superpose a picture currently displayed on the transmissive display (38) onto the image provided by the optical system (18), which, in operation is directed onto at least a section of the object (4).

10. Microscope according to claim 9, comprising a visible light-camera (28), another part (26) of the light (22) being directed to the visible-light camera (28) by the at least one beam splitter system (24).

11. Method for displaying microscope images, comprising the steps of
receiving, by an input interface (48), subsequent sets (50) of input image data (52),
receiving, by the input interface (48), auxiliary input data (58),
generating, by an image processor (80), subsequent sets of pseudo-color image data (86) in real time depending on the content of at least one of the auxiliary input data (58) and the input image data (52),
merging, by the image processor (80), each set (84) of the pseudo-color image data (86) with each set of the input image data (52) to obtain a set (92) of three-dimensional output image data (94),
wherein the pseudo-color image data (86) are generated at different depth layers of the three-dimensional output image data depending on the content of the auxiliary input data (58),
wherein a pseudo-color pattern field (88) is generated, by the image processor (80), within the pseudo-color image data (86) depending on the content of at least one of the input image data (52), the auxiliary input data (58), and the pseudo-color image data (86), wherein the pseudo-color pattern field (88) comprises a temporally modulated pattern (87",87"') having a variation rate that is computed depending on the content of at least one of the input image data (52) and the auxiliary input data (58).
and displaying the output image data (94) at an output data rate.

12. Method according to claim 11, wherein at least one set of the auxiliary input data are received from an ultrasound sensor, a microradiography camera, a thermographic camera, a fluorescence camera, multispectral camera, and a gamma-ray camera.

13. Computer program comprising instructions which when carried out by a computer cause said computer to carry out the method according to claim 11 or 12.

## Patentansprüche

1. Mikroskop, umfassend eine Eingangsschnittstelle (48) und eine Ausgangsschnittstelle (90), wobei die Eingangsschnittstelle (48) so konfiguriert ist, dass sie aufeinanderfolgende Sätze (50) von Eingangsbilddaten (52) und Hilfseingangsdaten (58) empfängt, wobei die Ausgangsschnittstelle (91) so konfiguriert ist, dass sie aufeinanderfolgende Sätze (92) von Ausgangsbilddaten (94) mit einer Ausgangsdatenrate ausgibt, wobei das Mikroskop weiter einen Bildprozessor (80) umfasst, wobei der Bildprozessor mit der Eingangsschnittstelle (48) zum Empfangen der aufeinanderfolgenden Sätze (50) der Eingangsbilddaten (52) und der Hilfseingangsdaten (58) und mit der Ausgangsschnittstelle (91) zum Ausgeben der aufeinanderfolgenden Sätze (92) der Ausgangsbilddaten (94) verbunden ist, wobei der Bildprozessor weiter so konfiguriert ist, dass er in Echtzeit aufeinanderfolgende Sätze (84) von Pseudofarbbilddaten (86) in Abhängigkeit von einem Inhalt von mindestens einem von den Hilfseingangsdaten (58) und den Eingangsbilddaten (52) erzeugt, wobei die Ausgangsbilddaten (94) dreidimensional sind und wobei der Bildprozessor (80) so konfiguriert ist, dass er die Pseudofarbbilddaten (86) in verschiedenen Tiefenschichten der dreidimensionalen Ausgangsbilddaten in Abhängigkeit von dem Inhalt der Hilfseingangsdaten (58) erzeugt, wobei der Bildprozessor (80) weiter so konfiguriert ist, dass er ein synthetisches Pseudofarbmusterfeld (88) innerhalb der Pseudofarbbilddaten (86) in Abhängigkeit von dem Inhalt von mindestens einem von den Eingangsbilddaten (52), den Hilfseingangsdaten (58) und den Pseudofarbbilddaten (86) erzeugt, wobei das Pseudofarbmusterfeld (88) ein zeitlich moduliertes Muster (87", 87"') mit einer Variationsrate umfasst, und wobei der Bildprozessor so konfiguriert ist, dass er die Variationsrate des zeitlich modulierten Musters (87", 87"') in Abhängigkeit von dem Inhalt von mindestens einem von den Eingangsbilddaten (52) und den Hilfseingangsdaten (58) berechnet, wobei der Bildprozessor (80) so konfiguriert ist, dass er die Pseudofarbbilddaten (86) und mindestens einen Abschnitt der Eingangsbilddaten (52) zusammenführt, um die Ausgangsbilddaten (94) zu erzeugen, die das Pseudofarbmusterfeld (88) aufweisen.

2. Mikroskop nach Anspruch 1, wobei das Pseudofarbmusterfeld (88) weiter ein sich räumlich änderndes Pseudofarbmuster (87, 87') umfasst.

3. Mikroskop nach Anspruch 2, wobei aufeinanderfolgende Sätze (50) der Ausgangsbilddaten (52) das Pseudofarbmusterfeld (88) umfassen, und wobei das zeitlich modulierte Muster ein sich ausbreitendes Wellenmuster (87b) umfasst, von dem mindestens eines von einer Geschwindigkeit, einer Richtung und einer Wellenlänge von dem Inhalt der Hilfseingangsdaten (58) abhängt.

4. Mikroskop nach einem der Ansprüche 1 bis 3, wobei die Ausgangsbilddaten (52) das Pseudofarbmusterfeld (88) in einem Bereich aufweisen, in dem die Inhalte der Hilfseingangsdaten (58) mindestens entweder unterhalb oder oberhalb eines Schwellenwertes liegen.

5. Mikroskop nach einem der Ansprüche 1 bis 4, wobei die Hilfseingangsdaten (58) dreidimensionale Hilfseingangsdaten (66) umfassen und wobei der Bildprozessor (80) so angepasst ist, dass er das Pseudofarbmusterfeld (88) in Abhängigkeit von dem Inhalt der dreidimensionalen Hilfseingangsdaten (67) erzeugt.

6. Mikroskop nach einem der Ansprüche 1 bis 5, umfassend eine Kamera (28) für sichtbares Licht, die mit der Eingangsschnittstelle (48) verbunden ist, um die aufeinanderfolgenden Sätze (50) der Eingangsbilddaten (52) bereitzustellen, und umfassend mindestens eines von einer Fluoreszenzkamera (32) und einem Ultraschallsensor (40, 42), die mit der Eingangsschnittstelle (48) verbunden sind, um die Hilfseingangsdaten (58) bereitzustellen.

7. Mikroskop nach Anspruch 6, umfassend die Fluoreszenzkamera (32) und eine Lichtquelle, wobei mindestens eines von der Fluoreszenzkamera (32), der Lichtquelle (8) und der Kamera (28) für sichtbares Licht mit einer Filteranordnung (44) für mindestens zwei verschiedene Fluorophore (14, 16) versehen ist, wobei jedes Fluorophor (14, 16) verschiedene Spitzenemissionswellenlängen aufweist, wobei die Ausgangsbilddaten (94) mindestens zwei Pseudofarben (89) umfassen und der Bildprozessor (80) so konfiguriert ist, dass er jeder der verschiedenen Fluoreszenzspitzenemissionswellenlängen eine verschiedene Pseudofarbe (89) zuordnet.

8. Mikroskop nach Anspruch 6 oder 7, weiter umfassend den Ultraschallsensor (40, 42), wobei der Bildprozessor (80) konfiguriert ist nach einem der Ansprüche 2 bis 6 und weiter so konfiguriert ist, dass er das Pseudofarbmusterfeld (88) in den Ausgangsbilddaten (94) in Abhängigkeit von dem Inhalt der Hilfseingangsdaten (58) von dem Ultraschallsensor (40, 42) erzeugt.

9. Mikroskop nach einem der Ansprüche 1 bis 8, umfassend ein optisches System (18), eine transmissive Anzeige (38), ein Okular (36) und mindestens ein Strahlenteilersystem (24), wobei die transmissive Anzeige (38) zwischen dem Okular (36) und dem mindestens einen Strahlenteilersystem (24) angeordnet ist, wobei das Okular (36) auf die transmissive Anzeige (38) gerichtet ist, wobei die transmissive Anzeige (38) so konfiguriert ist, dass sie einen Teil (34) des Lichts (22) von einem untersuchten Objekt (4) durchlässt und ein aktuell auf der transmissiven Anzeige (38) angezeigtes Bild auf das von dem optischen System (18) bereitgestellte Bild legt, das in dem Betrieb auf mindestens einen Abschnitt des Objekts (4) gerichtet ist.

10. Mikroskop nach Anspruch 9, umfassend eine Kamera (28) für sichtbares Licht, wobei ein anderer Teil (26) des Lichts (22) durch das mindestens eine Strahlenteilersystem (24) auf die Kamera (28) für sichtbares Licht gerichtet wird.

11. Verfahren zum Anzeigen von Mikroskopbildern, umfassend die Schritte von Empfangen, durch eine Eingangsschnittstelle (48), von aufeinanderfolgenden Sätzen (50) von Eingangsbilddaten (52), Empfangen, durch die Eingangsschnittstelle (48), von Hilfseingangsdaten (58), Erzeugen, durch einen Bildprozessor (80), von aufeinanderfolgenden Sätzen von Pseudofarbbilddaten (86) in Echtzeit in Abhängigkeit von dem Inhalt von mindestens einem von den Hilfseingangsdaten (58) und den Eingangsbilddaten (52), Zusammenführen, durch den Bildprozessor (80), jedes Satzes (84) der Pseudofarbbilddaten (86) mit jedem Satz der Eingangsbilddaten (52), um einen Satz (92) von dreidimensionalen Ausgangsbilddaten (94) zu erhalten, wobei die Pseudofarbbilddaten (86) in verschiedenen Tiefenschichten der dreidimensionalen Ausgangsbilddaten in Abhängigkeit von dem Inhalt der Hilfseingangsdaten (58) erzeugt werden, wobei ein Pseudofarbmusterfeld (88) durch den Bildprozessor (80) innerhalb der Pseudofarbbilddaten (86) in Abhängigkeit von dem Inhalt von mindestens einem von den Eingangsbilddaten (52), den Hilfseingangsdaten (58) und den Pseudofarbbilddaten (86) erzeugt wird, wobei das Pseudofarbmusterfeld (88) ein zeitlich moduliertes Muster (87", 87"') mit einer Variationsrate umfasst, die in Abhängigkeit von dem Inhalt von mindestens einem von den Eingangsbilddaten (52) und den Hilfseingangsdaten (58) berechnet wird und Anzeigen der Ausgangsbilddaten (94) mit einer Ausgangsdatenrate.

12. Verfahren nach Anspruch 11, wobei mindestens ein Satz der Hilfseingangsdaten von einem Ultraschallsensor, einer Mikroradiographiekamera, einer Thermographiekamera, einer Fluoreszenzkamera, einer Multispektralkamera und einer Gammastrahlenkamera empfangen wird.

13. Computerprogramm, umfassend Anweisungen, die, wenn sie von einem Computer ausgeführt werden, den Computer veranlassen, das Verfahren nach Anspruch 11 oder 12 auszuführen.

## Revendications

1. Microscope, comprenant une interface d'entrée (48) et une interface de sortie (90),
l'interface d'entrée (48) étant destinée à recevoir des ensembles ultérieurs (50) de données d'image (52) d'entrée et de données d'entrée auxiliaires (58),
l'interface de sortie (91) étant destinée à sortir des ensembles (92) ultérieurs de données d'image (94) de sortie à un débit de données de sortie,
le microscope comprenant en outre un processeur d'image (80),
dans lequel le processeur d'image est connecté à l'interface d'entrée (48) pour recevoir les ensembles ultérieurs (50) des données d'image (52) d'entrée et des données d'entrée auxiliaires (58), ainsi qu'à l'interface de sortie (91) pour sortir les ensembles (92) ultérieurs des données d'image (94) de sortie,
le processeur d'image étant en outre destiné à générer, en temps réel, des ensembles (84) ultérieurs de données d'image en pseudo-couleur (86) en fonction d'un contenu d'au moins une des données d'entrée auxiliaires (58) et des données d'image (52) d'entrée,
dans lequel les données d'image (94) de sortie sont tridimensionnelles et
dans lequel le processeur d'image (80) est destiné à générer les données d'image en pseudo-couleur (86) à des couches de profondeurs différentes des données d'image de sortie tridimensionnelles en fonction du contenu des données d'entrée auxiliaires (58),
dans lequel le processeur d'image (80) est en outre destiné à générer un champ de motif en pseudo-couleur (88) synthétique à l'intérieur des données d'image en pseudo-couleur (86) en fonction du contenu d'au moins une des données d'image (52) d'entrée, des données d'entrée auxiliaires (58) et des données d'image en pseudo-couleur (86), le champ de motif en pseudo-couleur (88) comprenant un motif modulé temporellement (87", 87"') présentant un taux de variation, et
dans lequel le processeur d'image est destiné à calculer le taux de variation du motif modulé temporellement (87", 87‴) en fonction du contenu d'au moins une des données d'image (52) d'entrée et des données d'entrée auxiliaires (58),
dans lequel le processeur d'image (80) est destiné à fusionner les données d'image en pseudo-couleur (86) et au moins une section des données d'image (52) d'entrée afin de générer les données d'image (94) de sortie contenant le champ de motif en pseudo-couleur (88).

2. Microscope selon la revendication 1,
dans lequel le champ de motif en pseudo-couleur (88) comprend en outre un motif en pseudo-couleur changeant spatialement (87, 87').

3. Microscope selon la revendication 2,
dans lequel des ensembles ultérieurs (50) des données d'image (52) de sortie comprennent le champ de motif en pseudo-couleur (88), et dans lequel le motif modulé temporellement comprend un motif d'onde de propagation (87b) dont au moins un élément parmi une vitesse, une direction et une longueur d'onde dépend du contenu des données d'entrée auxiliaires (58).

4. Microscope selon l'une quelconque des revendications 1 à 3,
dans lequel les données d'image (52) de sortie
contiennent le champ de motif en pseudo-couleur (88) dans une zone où les contenus des données d'entrée auxiliaires (58) sont au moins une zone parmi une zone au-dessous d'un seuil et au-dessus d'un seuil.

5. Microscope selon l'une quelconque des revendications 1 à 4,
dans lequel les données d'entrée auxiliaires (58)
comprennent des données d'entrée auxiliaires tridimensionnelles (66) et dans lequel le processeur d'image (80) est adapté à générer le champ de motif en pseudo-couleur (88) en fonction du contenu des données d'entrée auxiliaires tridimensionnelles (67).

6. Microscope selon l'une quelconque des revendications 1 à 5,
comprenant une caméra à lumière visible (28)
connectée à l'interface d'entrée (48) pour fournir les ensembles ultérieurs (50) des données d'image (52) d'entrée et comprenant au moins un élément parmi une caméra à fluorescence (32) et un capteur ultrasonore (40, 42) connecté à l'interface d'entrée (48) pour fournir les données d'entrée auxiliaires (58).

7. Microscope selon la revendication 6,
comprenant la caméra à fluorescence (32) et une source de
lumière (8), au moins un élément parmi la caméra à fluorescence (32), la source de lumière (8) et la caméra à lumière visible (28) étant muni d'un agencement de filtre (44) pour au moins deux fluorophores (14, 16) différents, chaque fluorophore (14, 16) présentant des longueurs d'onde d'émission de pic différentes, les données d'image (94) de sortie comprenant au moins deux pseudo-couleurs (89) et le processeur d'image (80) étant configuré pour attribuer une pseudo-couleur (89) différente à chacune des longueurs d'onde d'émission de pic de fluorescence différentes.

8. Microscope selon la revendication 6 ou 7,
comprenant en outre le capteur ultrasonore (40, 42),
dans lequel le processeur d'image (80) est configuré selon l'une quelconque des revendications 2 à 6 et en outre destiné à générer le champ de motif en pseudo-couleur (88) dans les données d'image (94) de sortie
en fonction du contenu des données d'entrée auxiliaires (58) en provenance du capteur ultrasonore (40, 42).

9. Microscope selon l'une quelconque des revendications 1 à 8,
comprenant un système optique (18), un
afficheur transmissif (38), un oculaire (36) et au moins un système séparateur de faisceau (24), l'afficheur transmissif (38) étant agencé entre l'oculaire (36) et l'au moins un système séparateur de faisceau (24), l'oculaire (36) étant dirigé sur l'afficheur transmissif (38),
l'afficheur transmissif (38) étant destiné à permettre à une partie (34) de lumière (22) en provenance d'un objet (4) inspecté de passer et de se superposer à une image actuellement affichée sur l'afficheur transmissif (38) sur l'image fournie par le système optique (18), qui, en fonctionnement, est dirigée sur au moins une section de l'objet (4).

10. Microscope selon la revendication 9, comprenant
une caméra à lumière visible (28), une autre partie (26) de la lumière (22) étant dirigée vers la caméra à lumière visible (28) par l'au moins un système séparateur de faisceau (24).

11. Procédé destiné à afficher des images de microscope, comprenant les étapes suivantes
recevoir, par une interface d'entrée (48), des ensembles ultérieurs (50) de données d'image (52) d'entrée,
recevoir, par l'interface d'entrée (48), des données d'entrée auxiliaires (58),
générer, par un processeur d'image (80),
des ensembles ultérieurs de données d'image en pseudo-couleur (86) en temps réel en fonction du
contenu d'au moins une des données d'entrée auxiliaires (58) et des données d'image (52) d'entrée,
fusionner, par le processeur d'image (80),
chaque ensemble (84) des données d'image en pseudo-couleur (86) avec chaque ensemble des données d'image (52)
d'entrée pour obtenir un ensemble (92) de données d'image (94) de sortie tridimensionnelles,
dans lequel les données d'image en pseudo-couleur (86) sont générées à des couches de profondeurs différentes des données d'image de sortie tridimensionnelles en fonction du contenu des données d'entrée auxiliaires (58),
dans lequel un champ de motif en pseudo-couleur (88) est généré, par le processeur d'image (80), à l'intérieur des données d'image de pseudo-couleur (86) en fonction du contenu d'au moins une des données d'image (52) d'entrée, des données d'entrée auxiliaires (58) et des données d'image en pseudo-couleur (86),
dans lequel le champ de motif en pseudo-couleur (88) comprend un motif modulé temporellement (87",87‴) présentant un taux de variation qui est calculé en fonction du contenu d'au moins une des données d'image (52) d'entrée et des données d'entrée auxiliaires (58),
et afficher les données d'image (94) de sortie à un débit de données de sortie.

12. Procédé selon la revendication 11,
dans lequel au moins un ensemble des données d'entrée auxiliaires est
reçu en provenance d'un capteur ultrasonore, d'une caméra de microradiographie, d'une caméra de thermographie, d'une caméra à fluorescence, d'une caméra multispectrale et d'une caméra à rayons gamma.

13. Programme d'ordinateur comprenant des instructions qui, lorsqu'elles sont réalisées par un ordinateur, amènent ledit ordinateur à réaliser le procédé selon la revendication 11 ou 12.
